# EUROPEAN PATENT SPECIFICATION

(11) **EP 0 427 444 B1**
(45) Date of publication and mention of the grant of the patent: **07.04.1993**
(21) Application number: 90311900.6
(22) Date of filing: 30.10.1990
(51) Int. Cl.: A61F 2/30

(54) **Centraliser for the stem of an intramedullary prosthesis**
Vorrichtung zum Zentralisieren des Stieles einer intramedulären Prothese
Centralisateur pour la tige d'une prothèse intramédullaire

(30) Priority: 06.11.1989 GB 8924985
(43) Date of publication of application: 15.05.1991
(73) Proprietor: HOWMEDICA INTERNATIONAL INC., Shannon Co. Clare (IE)
(72) Inventor: Ashby, Alan Miles, Maidenhead, Berkshire SL6 1EB (GB)
(74) Representative: Bridge-Butler, Alan James

(56) References cited:
- EP-A- 0 006 408
- EP-A- 0 220 427
- GB-A- 1 409 053
- GB-A- 2 052 267
- GB-A- 2 104 391

## Description

This invention relates to a centraliser for the stem of an intramedullary prosthesis.

Devices of this kind are known, for example as shown in British Patent Application 2 104 391. A disadvantage with devices of this kind is that the resilient fingers which are provided to centralise the tip end of the stem of the prosthesis can only be pressed inwardly to a certain extent and difficulties can arise if the intramedullary canal is particularly narrow. It will be appreciated that the spacer is not only to centralise the tip end of the prosthesis but also to prevent it actually engaging a wall of the canal.

A further disadvantage with devices of this kind is that they tend to leave voids and air bubbles in the cement behind them as they are pushed downwardly and these voids and air bubbles also form behind the fingers, that is between the fingers and the body of the device. This is to be avoided as it can become a point of weakness and crack initiation within the cement mantel.

The present invention is intended to overcome some of the disadvantages referred to above.

It is also advantageous to provide a centraliser on which in certain circumstances the forces causing subsidence, such as a heavy load on the prosthesis are high enough to cause the stem to move further into the cement mantle, and thus further into the centraliser, which offers the advantage of being able to absorb this movement by providing means to accept the movement cover the end of the stem to thus prevent it piercing further into the cement and causing cracking.

According to the present invention a centraliser for the stem of a prosthesis for introduction into an intramedullary canal comprises an annular body portion for location on the tip end of said stem and provided with three or more resiliently deformable fins or wings projecting outwardly therefrom and which are adapted to fold circumferentially and inwardly towards said body portion.

Thus, with this construction the fins are deflected inwardly towards the body portion as the stem enters the narrower part of a canal. In addition due to the fact that the fins can be made of relatively thin section they cut their way through the bone cement as the stem is introduced without having to bulldoze their way through the cement leaving voids in their wake. Thus, voids and air bubbles are prevented or reduced.

A further advantage is that these thin fins or wings can collapse inwardly down to a diameter of less than 10 mm and therefore the surgeon will less frequently be faced with the need to trim the guiding means off the central tube. This extra step has sometimes been necessary with constructions as set forth in the earlier Patent Application referred to above.

Preferably the fins or wings extend downwardly from the said body portion in tangential directions so that they are lined up ready for deformation in the correct direction.

The body portion can be provided with control means to allow the tip end to move further into it subsequently to fitting and after the prosthesis and centraliser have been cemented into place.

With this arrangement the body portion is preferably shaped to enclose the tip end of the prosthesis stem.

Thus, the body portion can be in the form of a cup one end of which is closed and the control means are provided by a deformable control collar which can be at a mid point or at the open end of the cup.

Thus, the tip end is always enclosed even after some subsidence of the prosthesis has taken place without the tip contorting as resistance to this subsidence.

Alternatively the centraliser can be provided in combination with separate enclosure means for housing the tip end of said stem and which are provided with control means for allowing the tip to move further into said enclosure means subsequently to fitting.

The enclosure means can be provided in the form of a cup portion one end of which is closed and the control means are in the form of a collar at a mid point or at the other end of a cup.

The centraliser and/or the enclosure means can be made from a material similar to bone cement material which will thus enable it to become well integrated with the cement mantle and avoid any weakening or hole creation. Thus, it can be made from polymethylmethacrylate to which a filler has been added to provide a little more flexibility. The bone cement will, creep so that after becoming integrated with the centraliser the control means can continue to function.

The invention also includes a centraliser and/or enclosure means as set forth above in combination with a prosthetic device, for example the stem of a hip or knee replacement prosthesis.

The invention can be performed in various ways and some embodiments will now be described by way of example and with reference to the accompanying drawings in which :-
Figure 1 is a cross-sectional side elevation of a centraliser according to the invention;
Figure 2 is a plan view of the centraliser shown in Figure 1;
Figure 3 is a plan view of an alternative construction;
Figure 4 is a side view partly in section of an alternative construction of centraliser mounted in combination with enclosure means on the stem of an intramedullary prosthesis;
Figure 5 is a plan view of the centraliser shown in Figure 4; and
Figures 6 and 7 illustrate an alternative construction.

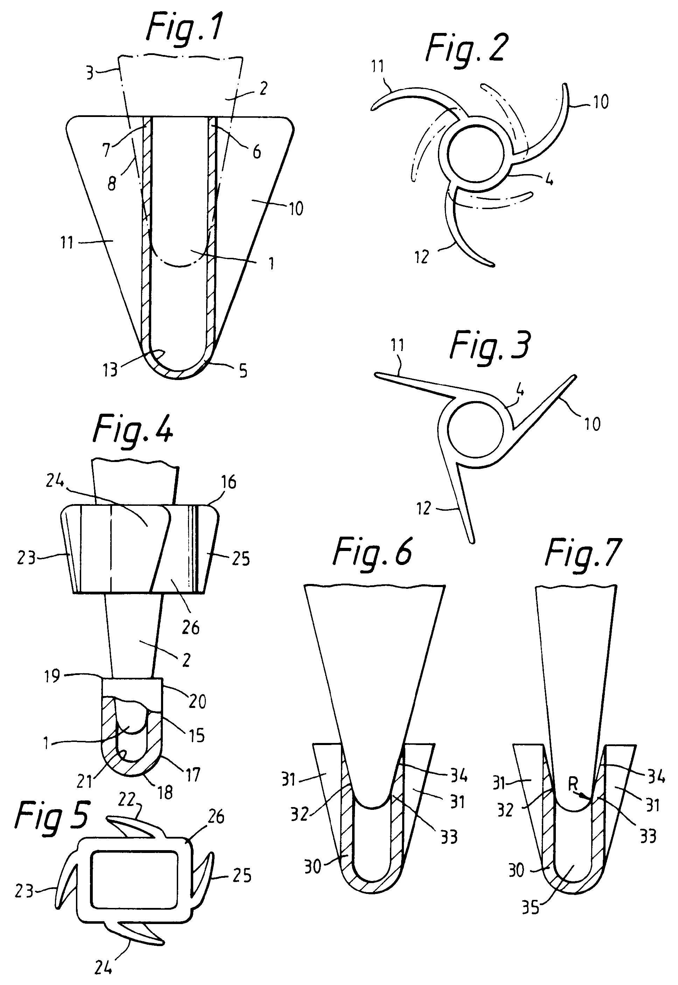

As shown in Figure 1 the centraliser comprises means for centralising a prosthesis and at the same time includes means for accommodating subsequent subsidence of the stem of the prosthesis into a polymerised, cured or set cement mantle in a medullary canal after surgical implantation.

The device acts to locate the tip end 1 of the stem 2 of a prosthetic hip device indicated by broken lines 3 by locating it within the medullary canal in which it is fitted to prevent contact between the stem of the hip device and the bone and to act to accommodate the subsidence referred to.

The centraliser comprises a body portion 4 in the form of a cup having a lower closed end 5 and an upper open end 6. This upper end 6 of the cup is provided as a deformable collar 7. In the drawing the collar is shown in the unengaged position but when pushed onto a prosthesis it will tend to deform outwardly to the position shown in broken lines 8.

Means for holding the stem 1 away from the wall of a medullary canal are provided by three equally spaced tangentially projecting arms 10, 11, 12.

The dimensions of the control collar 7 are arranged so that when the centraliser is pushed onto the tip of the prosthesis it deforms slightly into the position shown in broken lines 8 where it is held in position. The prosthesis is now fitted by pushing it into the medullary canal which has been suitably prepared and which contains the necessary cement, because the cement has not yet polymerised, cured or set, the forces are not sufficient to force the collar 7 all the way up stem tip 1. The prosthesis is inserted in the appropriate place, the arms 10, 11, 12 deforming inwardly to ensure that there is correct location. A typical deformed position of the arms is shown in broken lines in Figure 2.

The central collar 7 acts as a seal around the stem 1 and is effective to maintain a small void distal to the stem tip.

In the arrangement described the centraliser is moulded from polymethylmethacrylate to which a filler has been added to provide more flexibility and so that the arms 10, 11, 12 are resiliently deformable as is the control collar 7.

With the centraliser in position the polymethylmethacrylate centraliser becomes well integrated with the cement which can be of similar material without the flexible filler thus avoiding any weakening or hole creation.

When the prosthesis has been in use for some time it may have a tendency to sink further into the cement. This can be accommodated by the control collar deforming to allow the prosthesis to pass further into the cup 4. It will be appreciated this will considerably reduce the chances of the arms 10, 11, 12 breaking away or of the cup itself sinking further. The movement is merely accommodated by deformation of the cup and the surrounding cement mantle. Moreover, the tip of the prosthesis remains completely covered, the gap between the lower end of the prosthesis and the inner surface 13 of the lower end of the cup thus accommodating the movement.

A device of this kind can be used with any stem provided the inner dimensions of the cup are large enough and of suitable geometry to fit the end of the hip stem. The device however, is particularly appropriate for use with prostheses such as the "EXETER" prosthesis which has no collar at the, top. This type of prosthesis stem is designed on the principle of taper engagement between the metal stem and the cement. It is important that this taper can reengage if there is any, movement in the cement and in order to achieve this it is important that there is no cement around the distal tip of the stem.

Figure 3 shows an alternative construction in which similar reference numerals are used to indicate similar parts. In this construction however the fins or wings 10, 11, 12 are straight rather than curved although they are still tangential to the cup 4.

Once again a cup with three fins or wings is shown but it will be appreciated that four or five wings could be used if desired.

In the arrangement shown in Figures 4 and 5 separate enclosure means 15 are provided and which are separate from the centraliser 16 and as above is attached to the stem tip prior to insertion. The stem of the prosthesis is indicated by reference numeral 2 and its tip by reference numeral 1.

The enclosure means 15 is provided by a cup 17 having a lower closed end 18 and an upper open end 19. The upper end 19 of the cup portion is provided with a deformable collar 20. The inner surface of the lower end of the cup 17 is indicated by reference numeral 21.

The centraliser 16 can, for example, have a plan view similar to Figures 2 and 3 but in the arrangement shown comprises four equally spaced arms 22, 23, 24, and 25 which extend tangentially from a ring or collar 26. This ring acts as a control means to locate the centraliser on the prosthesis.

The centraliser and enclosure means can again be moulded from polymethylmethacrylate to which a filler has been added to provide more flexibility and so that the arms 22, 23, 24, 25 are resiliently deformable as is the collar 26.

The dimensions of the collar are arranged so that when the centraliser is pushed onto the tip of the prosthesis the ring deforms slightly so that it is held in position. The cup 18 is now pushed onto the tip of the prosthesis, control collar 20 deforming it to hold it in position.

The prosthesis is now fitted in the manner described above and the same process with regard to the cement and prosthesis sinking being as set forth and as described with regard to Figures 1, 2 and 3.

In the arrangements described above the formable control collar 7 is provided on the cup at its open upper end 6 and the cup has substantially parallel sides. With cylindrical internal geometry very slim stems might bottom out in the centraliser and for the fattest stems the centraliser may fall off the stem prior to insertion during surgery.

Figures 6 and 7 show an alternative construction which is more adaptable to stems of varying shapes and sizes. In this construction the centraliser comrpises a cup having a body portion 30 and tangentially projecting arms indicated by reference numeral 31. The upper portion of the inside surface of the cup is tapered, as shown at 32 so that an effective control collar 33 is provided at a point displaced from the upper end 34.

In the arrangement shown in Figure 7 which is for a narrow stem size the reference numeral R indicates the radius of the stem tip. The dimensions are arranged so that the radius of the bore 35 of the cup is the same as the radius of the stem tip.

By making the open upper end of the bore 35 tapered in the manner described it has been found that a single centraliser size can fit several sizes of stem. Thus, Figure 6 shows the use of a centraliser for a large stem size and Figure 7 illustrates the use of the same centraliser for use with a narrow stem size. In each case it will be seen that the portion of the internal surface of the bore adjacent the change in dimensions on the parallel side surfaces provides a control collar.

It will be appreciated that the same type of internal configuration and dimensions for the cup can be employed with the separate enclosure means 15 of the kind shown in Figure 4. The centraliser 16 in Figure 4 and Figure 5 can have a straight sided internal bore or it could be tapered from one end to the other or again, alternatively, it could have reversed tapers or a taper at one end and be substantially parallel at the other, and as will be seen from Figure 5 the cross-section of the internal bore can be appropriate to the stem with which it is to be used, for example, it can be substantially rectangular triangular, circular or any other suitable shape.

It will be appreciated that due to the material which is used the parts become completely integrated with the cement thus avoiding any weakening or hole creation but the inward, movement of the prosthesis can be accommodated. The use of inwardly folding fins or wings allows the prosthesis to be used in narrow canals but more particularly is advantageous in as much that when the prosthesis is inserted into the cement the production of voids, gaps and holes behind the fins or wings is obviated.

## Claims

1. A centraliser for the stem (2) of a prosthesis (3) for introduction into an intramedullary canal comprising an annular body portion (4) for location on the tip (1) end of said stem (2) and provided with three or more resiliently deformable fins or wings (10,11,12) projecting outwardly therefrom, characterised in that the fins or wings are adapted to fold circumferentially and inwardly towards said body portion (4).

2. A centraliser as claimed in claim 1, in which said fins or wings (10,11,12) extend outwardly from said body portion (4) in tangential directions.

3. A centraliser as claimed in claim 1 or claim 2, in which said body portion (4) is provided with control means (7) to allow said tip end (1) to move further into it subsequently to fitting.

4. A centraliser as claimed in claim 3, in which said body portion (4) is shaped to enclose the tip end (1) of said prosthesis (3).

5. A centraliser as claimed in claim 4, in which said body portion (4) is in the form of a cup one end (5) of which is closed and the control means (7) are provided by a deformable control collar (7) at the open end (6) of the cup.

6. A centraliser as claimed in claim 3, in combination with enclosure means (15) for housing the tip end (1) of said stem (2) and which are provided with control means (20) for allowing the tip (1) to move further into said enclosure means (15) subsequently to fitting.

7. A centraliser as claimed in claim 6, in which said enclosure means (15) are in the form of a cup portion, one end (18) of which is closed and the control means (20) are in the form of a collar (20) at the other end (19) of the cup.

8. A centraliser as claimed in any of the preceding claims, in which the centraliser and/or enclosure means (15) are made from bone cement.

9. A centraliser as claimed in claim 8, made from polymethylmethacrylate to which a filler has been added.

10. A centraliser and/or enclosure means (15) as set forth in any of the preceding claims in combination with a prosthetic device.

## Patentansprüche

1. Vorrichtung zum Zentrieren des Stieles (2) einer Prothese (3) zur Einführung in einen intramedullären Kanal, umfassend ein ringförmiges Rumpfteil (4) zur Befestigung an der Spitze (1) des Stieles (2), welches mindestens drei nach außen hervorstehende federnd verformbaren Stege oder Flügel (10, 11, 12) aufweist, dadurch gekennzeichnet, daß die Stege oder Flügel sich nach innen entlang des Umfangs und in Richtung des Rumpfteils (4) biegen können.

2. Zentriervorrichtung nach Anspruch 1, bei welcher die Stege oder Flügel (10, 11, 12) sich von dem Rumpfteil (4) in tangentialen Richtungen nach außen erstrecken.

3. Zentriervorrichtung nach einem der Ansprüche 1 oder 2, bei welcher das Rumpfteil (4) eine Kontrollvorrichtung (7) aufweist, welche es der Spitze (1) erlaubt, sich nach der Einpassung weiter in dieses hineinzubewegen.

4. Zentriervorrichtung nach Anspruch 3, bei welcher das Rumpfteil (4) zur Aufnahme der Spitze (1) der Prothese (3) geformt ist.

5. Zentriervorrichtung nach Anspruch 4, bei welcher das Rumpfteil (4) die Form einer Tasse aufweist, deren eines Ende (5) geschlossen ist und die Kontrollvorrichtung (7) aus einem verformbaren Kontrollkragen (7) am offenen Ende (6) der Tasse besteht.

6. Zentriervorrichtung nach Anspruch 3 in Kombination mit einer Aufnahmevorrichtung (15) zur Aufnahme der Spitze (1) des Stiels (2), ausgestattet mit Kontrollvorrichtungen (20), welche es der Spitze (1) erlauben, sich nach dem Einpassen weiter in die Aufnahmevorrichtung (15) zu bewegen.

7. Zentriervorrichtung nach Anspruch 6, bei welcher die Aufnahmevorrichtung (15) die Form eines Tassenteils aufweist, dessen eines Ende (18) geschlossen ist und die Kontrollvorrichtung (20) als Kragen (20) am anderen Ende (19) der Tasse ausgebildet ist.

8. Zentriervorrichtung nach einem der voranstehenden Ansprüche, wobei die Zentriervorrichtung und/oder Aufnahmevorrichtung (15) aus Knochenzement hergestellt sind.

9. Zentriervorrichtung nach Anspruch 8, hergestellt aus Polymethylmethacrylat, welchem ein Füller beigefügt wurde.

10. Zentriervorrichtung und/oder Aufnahmevorrichtung (15) nach einem der voranstehenden Ansprüche in Kombination mit einer Prothese.

## Revendications

1. Centralisateur destiné à la tige (2) d'une prothèse (3) en vue de son introduction dans un canal intramédullaire, comportant une portion de corps annulaire (4) destinée à être placé sur l'extrémité de pointe (1) de ladite tige (2) et muni de trois ou plusieurs nervures ou ailettes (10, 11, 12) élastiquement déformables et en faisant saillie vers l'extérieur, caractérisé en ce que les nervures ou ailettes sont adaptées à s'infléchir circonférentiellement et vers l'intérieur en direction de ladite portion de corps (4).

2. Centralisateur selon la revendication 1, dans lequel lesdites nervures ou ailettes (10, 11, 12) s'étendent vers l'extérieur à partir de ladite portion de corps (4) dans des directions tangentielles.

3. Centralisateur selon la revendication 1 ou la revendication 2, dans lequel ladite portion de corps (4) est munie de moyens de commande (7) pour permettre à ladite extrémité de pointe (1) de s'avancer plus loin dans celle-ci après la mise en place.

4. Centralisateur selon la revendication 3, dans lequel ladite portion de corps (4) est conformée pour entourer l'extrémité de pointe (1) de ladite prothèse (3).

5. Centralisateur selon la revendication 4, dans lequel ladite portion de corps (4) a la forme d'une coupelle dont une extrémité (5) est fermée et les moyens de commande (7) sont constitués par un collier de commande déformable (7) à l'extrémité ouverte (6) de la coupelle.

6. Centralisateur selon la revendication 3 en combinaison avec des moyens d'entourage (15) pour loger l'extrémité de pointe (1) de ladite tige (2), qui sont munis de moyens de commande (20) pour permettre à la pointe (1) de s'avancer plus loin dans lesdits moyens d'entourage (15) après la mise en place.

7. Centralisateur selon la revendication 6, dans lequel lesdits moyens d'entourage (15) se présentent sous la forme d'une portion de coupelle dont une extrémité (18) est fermée et les moyens de commande (20) sont sous la forme d'un collier (20) à l'autre extrémité (19) de la coupelle.

8. Centralisateur selon l'une quelconque des revendications précédentes, dans lequel le centralisateur et/ou les moyens d'entourage (15) sont réalisés en un ciment à os.

9. Centralisateur selon la revendication 8, réalisé en polyméthacrylate de méthyle auquel a été ajoutée une charge.

10. Centralisateur et/ou moyens d'entourage (15) tels qu'indiqués dans l'une quelconque des revendications précédentes, en combinaison avec un dispositif prothétique.
